# EUROPEAN PATENT APPLICATION

(11) **EP 4 512 443 A1**
(43) Date of publication of application: **26.02.2025**
(21) Application number: 23207440.1
(22) Date of filing: 02.11.2023
(51) Int. Cl.: A61M 5/162, A61M 39/26

(54) **SNAP-ON CLOSED NEEDLELESS CONNECTOR MODULE FOR INFUSION SYSTEM AND INFUSION SYSTEM**

(30) Priority: 21.08.2023 TW 112131348
(71) Applicant: Tsai, Hsi-Chin, 238 New Taipei City (TW)
(72) Inventor: Tsai, Hsi-Chin, 238 New Taipei City (TW)
(74) Representative: Gee, Steven William

(57) **Abstract**

A snap-on closed needleless connector module for infusion system is provided, including a movable closed connector and a needleless connector. When the movable closed connector is separated from the needleless connector, the first snap-on part is separated from the second snap-on part, and the conduit is separated from the second sealing part, so that the slit of the second sealing part is tightly closed,; the elastic force of the second circumferential part resets the second sealing part to close the second opening, the first end of the housing is separated from the sleeve, and the positioning part and the first sealing part are reset by the elastic force of the first circumferential part, so that the second end of the first sealing part closes the first opening.

## Description

### BACKGROUND OF THE INVENTION

### 1. Field of the Invention

The present invention relates generally to a snap-on closed needleless connector module for infusion system and infusion system.

### 2. The Prior Arts

US Patent No. 10,688,295B2 disclosed a liquid transfer device for use with infusion liquid container, and the medicine mixing procedure includes the following steps:

According to the doctor's prescription, a vial bottle containing a certain powder additive is prepared; according to the doctor's prescription, an infusion liquid bag containing a diluting solution is prepared; the vial bottle is sheathed on the integral vial adapter of the needleless additive port, the puncturing member of the vial adapter punctures the IV spike of the vial bottle; one end of the puncturing member punctures the film of the infusion tube of the infusion liquid bag; the infusion liquid bag is on the top and the vial bottle is on the bottom. When the infusion liquid bag is squeezed, the diluting solution in the liquid infusion bag passes through the opening and channel of the puncturing part, the second flow channel of the output part, the first flow channel of the needleless additive port, and the puncturing member to enter the inside of the vial bottle; shake the vial bottle to slightly mix the powder in the vial bottle to obtain a high-concentration medicine; then put the liquid infusion bag at the bottom and the vial bottle at the top; squeeze the liquid infusion bag to release the air in the liquid infusion bag to pass through the through hole and long hole of the puncturing part, the second flow channel of the output part, the first flow channel of the needleless additive port and puncturing member to enter the inside of the vial bottle and release the squeezing hand to suck out the liquid inside the vial bottle, and repeat the operation until the liquid in the vial bottle is completely absorbed into the liquid infusion bag.

The infusion procedure for the conventional mixing device includes the following steps: step (a), remove the plug body of the administration port from the other end of the tube body; step (b), arrange the infusion drip set at the other end of the tube body and penetrate the sealing membrane, so that the infusion drip set can communicate with the second flow channel of the output part through the third flow channel of the tube body and; step (c), place the liquid infusion bag at the top and the infusion drip ser at the bottom so that the diluted medicine inside the liquid infusion bag passes through the through hole and long hole of the puncturing part, the second flow channel of the output part, the third flow channel of the tube body, and the infusion drip set in sequence to enter the patient's body.

However, when the high-concentration medicinal liquid in the vial bottle passes through the long hole of the puncturing part, due to the entry of air and the inability to absorb all the medicinal liquid at one time during suction, some high-concentration medicinal liquid will form residue attached to the long hole of the puncturing part. When the air in the liquid infusion bag passes through the long hole of the puncturing part, the air will push all the high-concentration medicinal liquid attached to the inner wall of the channel of the puncturing part to the space between the second flow channel and the insertion hole of the output part, and blocked by the tube body, forming residual high-concentration medicinal liquid. Even if the liquid infusion bag is loosened, the high-concentration medicinal liquid remaining in the space between the second flow channel of the output part and the insertion hole cannot be pushed into the liquid infusion bag by the flow of air. Therefore, when the chemical mixing process is repeated, the space between the second flow channel of the output part and the insertion hole will be filled with residual high-concentration medicinal liquid due to air squeezing.

Because the high-concentration medicinal liquid remaining in the space between the second flow channel of the output part and the insertion hole has not been diluted, the concentration is too high. Once the nursing staff uses the conventional medicine mixing device to perform the infusion procedure, the high-concentration medicinal liquid remaining in the space between the second flow channel of the output part and the insertion hole will first enter the patient's body, and the diluted medicinal liquid in the liquid infusion bag will then enters the patient's body. However, the entry of the residual high-concentration medicinal liquid into the patient's body will cause adverse reactions in the patient's body. Therefore, patients often report feeling very uncomfortable in the early stages of the infusion procedure. If this conventional medicine mixing device is used to infuse medicines into patients who require precise and quantitative administration, such as pregnant women or critically ill patients, serious consequences may occur, and even life-threatening consequences may occur.

Some nursing staff have noticed the above situation and will remove the residual in the space between the second flow channel of the output part and the insertion hole after step (a) of the infusion procedure and before step (b). High-concentration medicinal liquid is discharged from the third flow channel of the tube body. However, this practice directly leads to a reduction in the dose infused into the patient's body, which is inconsistent with the doctor's prescriptions, thereby reducing the therapeutic effect and resulting in medicine waste.

Furthermore, in step (c) of the infusion procedure of the conventional medicine mixing device, because the first flow channel of the needleless additive port and the second flow channel of the output part are still connected, part of the diluted medicine will flow from the second flow channel of the output part to enter the inside of the vial bottle through the first flow channel of the needleless additive port and the puncturing member, resulting in a reduction in the dose infused into the patient's body, which is inconsistent with the doctor's prescriptions, thereby reducing the therapeutic effect and resulting in medicine waste.

Moreover, because the needleless additive port has no sealing effect, the vial bottle cannot be removed from the needleless additive port, otherwise the residual high-concentration medicinal liquid will leak out through the needleless additive port. Therefore, the conventional medicine mixing device can only be used once and cannot be reused.

Furthermore, the conventional infusion system includes a medicine mixing device, an infusion drip set, a dispenser, a separator, and an retention needle. Because certain patients tend to move agitatedly, the dispenser and separator are easily separated, and the retention needle is easily separated from the artificial blood vessel, causing the medical solution and blood to leak out.

U.S. Patent No. 6,651,956B2 disclosed a slit-type swabable valve, U.S. Patent No. 9,415,199B2 disclosed a leak proof needleless medical connector, and U.S. Patent No. 10,201,693B2 disclosed a closed male luer. The above three patents can be disposed at the needleless additive port of the conventional needleless control valve shown in FIG. 1 of US 10,688,295B2 to be used for connecting the infusion drip set. The infusion drip set can deliver concentrated medicinal liquid into the liquid infusion bag through the above-mentioned patented combination medicine mixing device. The slit-type swabable valve has a sealing effect and solves the problem that the needleless additive port of US10,688,295B2 has no sealing effect. Therefore, the medicine mixing device of the above patent combination can be reused. Unfortunately, the above patent combination cannot solve other problems of US 10,688,295B2.

### SUMMARY OF THE INVENTION

A primary objective of the present invention is to provide a snap-on closed needleless connector module for infusion system and an infusion system thereof, which can prevent leakage of medicinal liquid.

In order to achieve the aforementioned objectives, the present invention provides a snap-on closed needleless connector module for infusion system, which includes a movable closed connector and a first needleless connector; wherein:
the movable closed connector comprising: a body, a first coupling part, and a first elastic valve; the body further comprising: a sleeve, a conduit, a positioning part and a first snap-on part; the conduit being arranged inside the sleeve, and having a first channel and a first opening; the positioning part being located inside the sleeve and disposed on the outside of the conduit; the first snap-on part being disposed on the sleeve, and the first coupling part being disposed on the sleeve and used for coupling a vial bottle, a syringe, or an infusion drip set of an infusion system; the first elastic valve comprising: a first circumferential part and a first sealing part, two ends of the first circumferential part being respectively arranged on the positioning part and the first coupling part, the first sealing part being located in the first channel, a first end of the first sealing part being connected to an inner side of the first circumferential part and disposed with a plurality of through holes, and the through holes communicating with the first channel, and a second end of the first sealing part closing the first opening;
the first needleless connector comprising: a housing, a second coupling part, and a second elastic valve; the housing being disposed with a second snap-on part, and the second snap-on part being disposed on an outer side of the housing; a first end of the housing having a second opening, a second coupling part being disposed at a second end of the housing, having a third opening, and being used for coupling a coupling seat of a medicine mixing device of the infusion system, an input end of a dispenser of the infusion system, or an input end of a separator of the infusion system; the second elastic valve comprising: a second circumferential part and a second sealing part, the second circumferential part being disposed inside the housing and the second coupling part and has a second channel, the second channel communicating with the third opening; the second sealing part being disposed inside the housing and having a slit, and the second sealing part closing the second opening;
wherein, when the movable closed connector is docked with the first needleless connector, the first snap-on part is fixed on the second snap-on part, the conduit compresses the second sealing part, and the second sealing part compresses the second circumferential part, so that the second sealing part is away from the second opening, and the conduit passes through the slit of the second sealing part to enter the inside of the second sealing part, the first end of the housing is against an inner wall of the sleeve, and the positioning part moves along conduit in a direction away from the first needleless connector and compresses the first circumferential part, so that the second end of the first sealing part is separated from the first opening, and the first channel communicates with the second channel; and
wherein, when the movable closed connector is separated from the first needleless connector, the first snap-on part is separated from the second snap-on part, and the conduit is separated from the second sealing part, so that the slit of the second sealing part is tightly closed, the second sealing part is reset by the elastic force of the second circumferential part and closes the second opening; the first end of the housing is separated from the sleeve, the positioning part and the first sealing part are reset by the elastic force of the first circumferential part, so that the second sealing part is reset by the elastic force of the first circumferential part so that the second end of the first sealing part closes the first opening .

In order to achieve the aforementioned objectives, the present invention provides an infusion system, including a first mixing device, at least a second mixing device, at least two infusion drip sets, a dispenser, a separator, and an retention needle; wherein:
the first mixing device comprising: a puncturing part, a needleless additive port, an output part, and a plug member; a first end of the puncturing part being a sharp end, and a U-shaped through hole and at least one side hole being disposed on one side wall of the puncturing part, a distance from the top of the U-shaped through hole to the sharp end of the puncturing part being less than a distance from the top of the at least one side hole to the sharp end of the puncturing part, and the puncturing part being divided by a first partition to divide the internal space into a first long hole and at least a second long hole; the first long hole communicating with the U-shaped through hole, and the at least one second long hole communicates with the at least one side hole; the needleless additive port comprising a coupling seat and an integral vial adaptor, the coupling seat being integrally formed on one side of the second end of the puncturing part, and disposed with a first flow channel; the first flow channel and the U-shaped through hole communicating with each other, the integral vial adaptor being integrally formed on the coupling seat, having a puncturing member, and being used for engaging with a vial bottle; the output part being disposed at the second end of the puncturing part, and being disposed with a second flow channel and an insertion hole; the second flow channel being connected between the at least one second long hole and the insertion hole, wherein a second partition being used between the needleless additive port and the output part to separate the first flow channel and the second flow channel, and the first partition being connected to the second partition; the plug member having a tube body, the tube body defining a third flow channel, and a first end of the tube body being inserted into insertion hole, so that the second flow channel communicating with the third flow channel, and a blocking part being disposed inside the third flow channel to close the third flow channel;
the at least one second medicine mixing device comprising: a puncturing part, a needleless additive port, an output part, a plug member, and a snap-on closed needleless connector module as described earlier, and a first end of the puncturing part being a sharp end; a U-shaped through hole and at least one side hole being disposed on one side wall of the puncturing part; a distance from the top of the U-shaped through hole to the sharp end of the puncturing part being less than a distance from the top of the at least one side hole to the sharp end of the puncturing part; the puncturing part being divided divides by a first partition to dividing internal space into a first long hole and at least a second long hole ; the first long hole and the U-shaped through holes communicating with each other, the at least one second long hole communicating with the at least one side hole; the needleless additive port comprising a coupling seat, the coupling seat being integrally formed on one side of the second end of the puncturing part and being disposed with a first flow channel, and the first flow channel communicating with the U-shaped through hole; the output part being disposed at the second end of the puncturing part and being disposed with a second flow channel and an insertion hole, the second flow channel communicating between the at least one second long hole and the insertion hole, wherein a second partition being disposed between the needleless additive port and the output part to separate the first flow channel and the second flow channel, and the first partition being connected to the second partition, the plug member having a tube body, the tube body defining a third flow channel, a first end of the tube body being inserted into the insertion hole, so that the second flow channel and the third flow channel channels communicating with each other, and a blocking part being disposed inside the third flow channel to close the third flow channel; the closed needleless connector module as mentioned above being defined as a first closed needleless connector module, and the first coupling part of the first closed needleless connector module being used for coupling to a vial bottle or a syringe, and the second coupling part of the first closed needleless connector module being used for coupling to the coupling seat;
a first end of one of the at least two infusion drip sets passing through the blocking part of the first mixing device, thereby allowing one of the infusion drip sets to communicate with the second flow channel through the third flow channel of the first medicine mixing device, and a first end of the other one of the infusion drip sets passing through the blocking part of the at least one second medicine mixing device, thereby allowing the other one of the infusion drip sets to communicate with the second flow channel through the third flow channel of the at least one second medicine mixing device;
the dispenser comprising: a gathering part, at least two input terminals, an output terminal, a closed needleless connector module as mentioned above, and a screw-type needleless connector module; the input terminals being connected to a first end of the gathering part, the output terminal being connected to a second end of the gathering part, the closed needleless connector module as mentioned above being defined as a second closed needleless connector module, the first coupling part of the second closed needleless connector module being used for coupling to a second end of one of the infusion drip sets, and the second coupling part of the second closed needleless connector module being used for coupling to one of the input terminals; the screw-type needleless connector module comprising a screw-coupling part and a second needleless connector; the screw-coupling part being disposed on the second end of sleeve of the other one of the infusion drip sets, a first end of the second needleless connector being screwed to the screw-coupling part, and a second end of the second needleless connector being disposed on the other one of the input terminals;
the separator comprising: a valve body, a screw-coupling structure, an output terminal, a third needleless connector, and a closed needleless connector module as mentioned above; an internal thread of a first end of the screw-coupling structure being screwed to an external thread of a first end of the valve body and defined as an input terminal of the separator; a first end of the output terminal being disposed on a second end of the valve body; the third needleless connector being disposed at a third end of the valve body; the closed needleless connector module as described above being defined as a third closed needleless connector module, and the second coupling part of the third closed needleless connector module being used for coupling to the screw-coupling structure; and
the retention needle being disposed at a second end of the output terminal of the separator.

The effect of the present invention is that both the movable closed connector and the first needleless connector have a sealing effect and can prevent leakage of the medicinal liquid.

### BRIEF DESCRIPTION OF THE DRAWINGS

The present invention will be apparent to those skilled in the art by reading the following detailed description of a preferred embodiment thereof, with reference to the attached drawings, in which:
FIG. 1 is a schematic diagram of the infusion system of the present invention.
FIG. 2 is a schematic diagram of the first medicine mixing device of the present invention.
FIG. 3 is a top view of the first medicine mixing device of the present invention.
FIG. 4 is an exploded view of the first embodiment of the second medicine mixing device of the present invention.
FIG. 5 is an exploded view of the second embodiment of the second medicine mixing device of the present invention.
FIG. 6 is an exploded view of the dispenser of the present invention.
FIG. 7 is an exploded view of the separator of the present invention.
FIG. 8 is a schematic diagram of the first medicine mixing device of the present invention performing medicine mixing and infusion.
FIG. 9 is a schematic diagram of the first embodiment of the second medicine mixing device of the present invention performing medicine mixing and infusion.
FIG. 10 is a schematic diagram of the second embodiment of the second medicine mixing device of the present invention performing mixing medicine and infusion.
FIG. 11 is a schematic diagram of the infusion of the dispenser of the present invention.
FIG. 12 is a schematic diagram of the infusion of the separator of the present invention.
FIG. 13 is a schematic diagram of the needleless connector of the present invention driving the screw-coupling structure to rotate or the needleless connector idling with respect to the screw-coupling structure.
FIG. 14 is a schematic diagram of the screw-coupling structure of the present invention being arbitrarily rotated along a first direction and a second direction with respect to the needleless connector.

### DETAILED DESCRIPTION OF THE PREFERRED EMBODIMENT

The accompanying drawings are included to provide a further understanding of the invention, and are incorporated in and constitute a part of this specification. The drawings illustrate embodiments of the invention and, together with the description, serve to explain the principles of the invention.

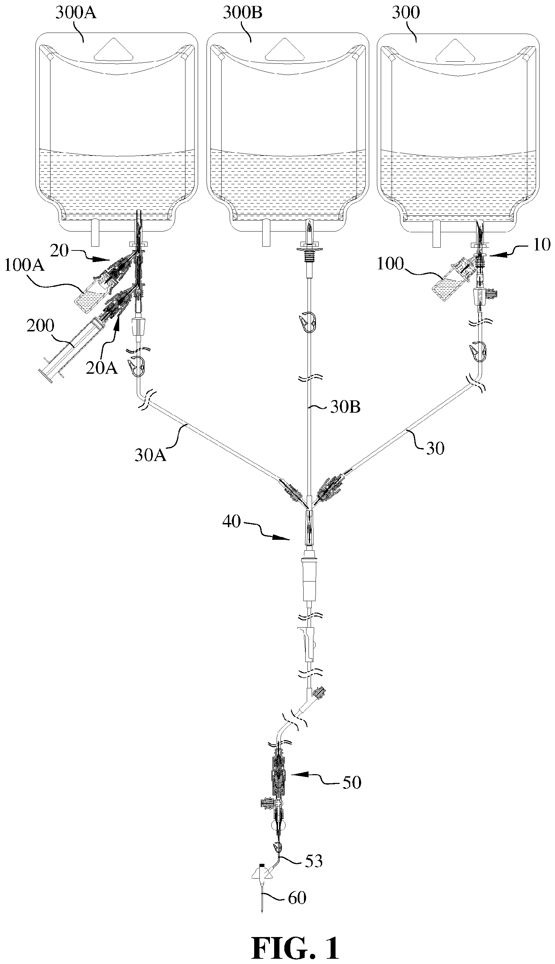

As shown in FIG. 1, the present invention provides an infusion system, including a first mixing device 10, two second mixing devices 20, 20A, three infusion drip sets 30, 30A, 30B, a dispenser 40, a separator 50, and a retention needle 60.

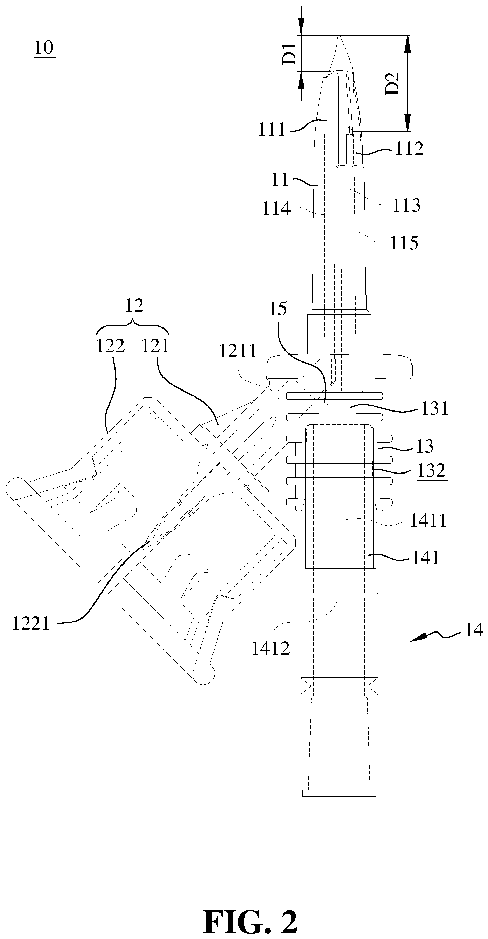

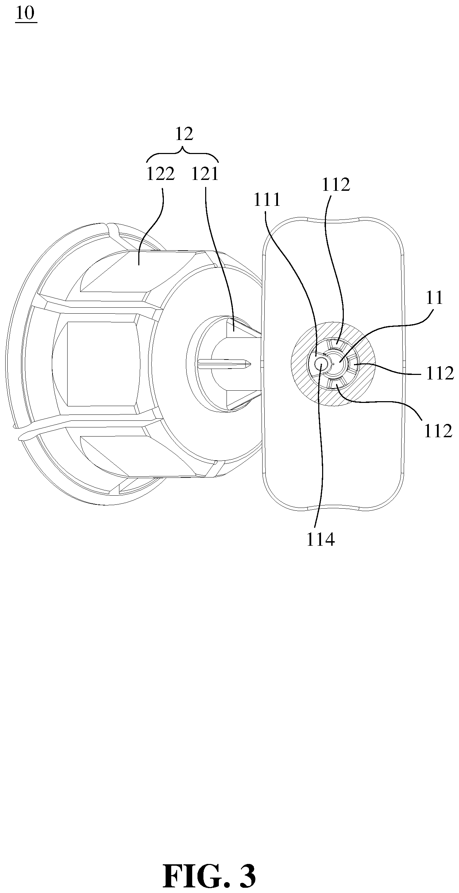

As shown in FIG. 2 and 3, the first medicine mixing device 10 includes a puncturing part 11, a needleless additive port 12, an output part 13, and a plug member 14.

A first end of the puncturing part 11 is a sharp end, and a U-shaped through hole 111 and three side holes 112 are disposed on one side wall of the puncturing part 11. The internal space of the puncturing part 11 is divided into a first long hole 114 and three second long holes 115 by a first partition 113. The first long hole 114 communicates with the U-shaped through hole 111, and each second long hole 115 communicates with each side hole 112. In addition, a distance D 1 from the top of the U-shaped through hole 111 to the sharp end of the puncturing part 11 is less than a distance D2 from the top of the side hole 112 to the sharp end of the puncturing part 11. In other words, the top of the U-shaped through hole 111 is closer to the sharp end of the puncturing part 11 and forms a U-shaped hole, and the top of the side hole 112 is farther from the sharp end of the puncturing part 11.

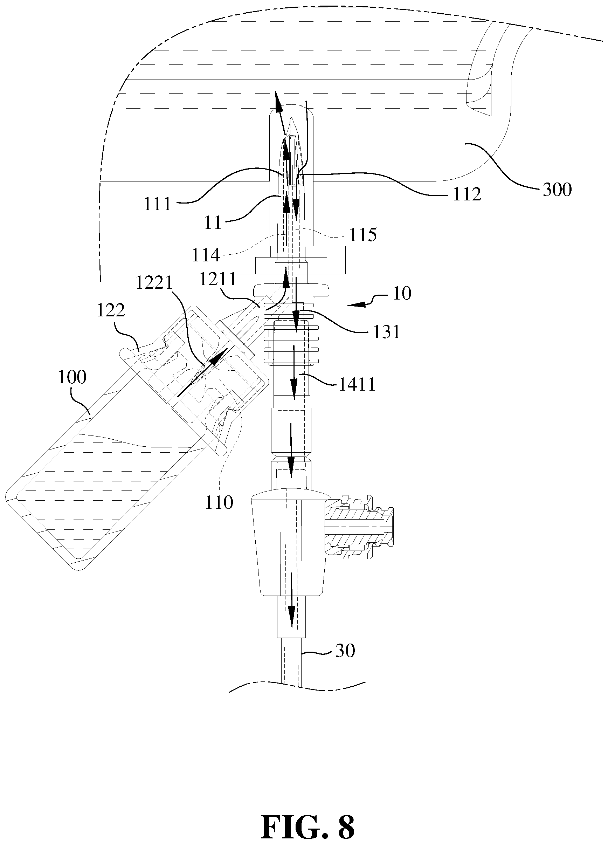

The needleless additive port 12 includes a coupling seat 121 and a integral vial adaptor122. The coupling seat 121 is integrally formed on one side of a second end of the puncturing part 11 and defines a first flow channel 1211, and the first flow channel 1211 communicates with the U-shaped through hole 111. The integral vial adaptor122 is integrally formed on the coupling seat 121, has a puncturing member 1221, and is used to be combined with a vial bottle 100 (see FIGS. 1 and 8).

The output part 13 is disposed at the second end of the puncturing part 11, and has a second flow channel 131 and a insertion hole 132. The second flow channel 131 is connected between the second long holes 115 and the insertion hole 132. In the present embodiment, the outer surface of the output part 13 is a smooth curved surface.

The first flow channel 1211 and the second flow channel 131 are separated by a second partition 15 between the needleless additive port 12 and the output part 13, and the first partition 113 is connected to the second partition 15.

The plug member 14 has a tube body 141. The tube body 141 defines a third flow channel 1411, and a first end of the tube body 141 is inserted into the insertion hole 132, so that the second flow channel 131 communicates with the third flow channel 1411. A blocking part 1412 is disposed inside the third flow channel 1411 to close the third flow channel 1411.

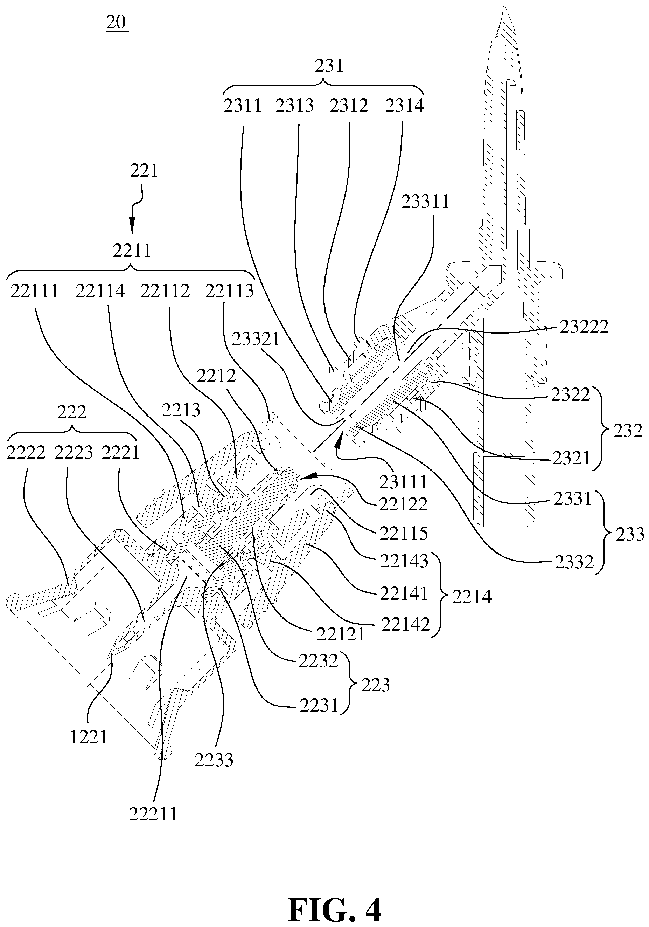

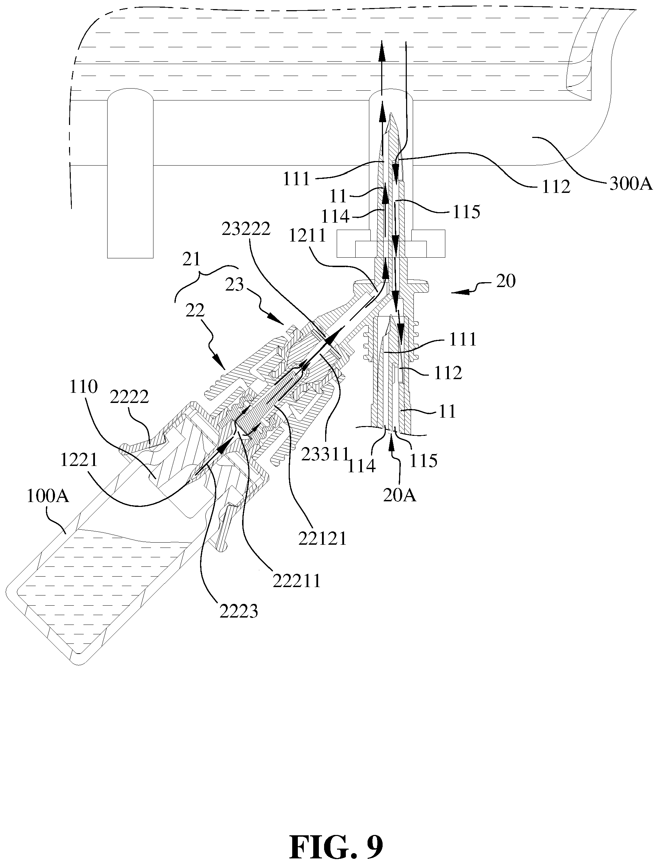

As shown in FIGS. 4 and 9, the structural difference between the second medicine mixing device 20 and the first medicine mixing device 10 is that the second medicine mixing device 20 further includes a first closed needleless connector module 21. The first closed needleless connector module 21 replaces the integral vial adaptor 122. Furthermore, the first closed needleless connector module 21 includes a movable closed connector 22 and a first needleless connector 23.

The movable closed connector 22 includes a body 221, a first coupling part 222, and a first elastic valve 223. The body 221 includes a sleeve 2211, a conduit 2212, a positioning part 2213 and a first snap-on part 2214. The sleeve 2211 includes an upper cylinder part 22111, a middle cylinder part 22112 and a lower cylinder part 22113. The middle cylinder part 22112 is disposed between a first end of the upper cylinder part 22111 and a first end of the lower cylinder part 22113. A first through hole 22114 is disposed between one side wall of the upper cylinder part 22111 and a side wall of the middle cylinder part 22112, and a second through hole is disposed between the side wall of the middle cylinder part 22112 and one side wall of the lower cylinder part 22113. 22115. The conduit 2212 extends from the inside of the upper cylinder part 22111 to the inside of the middle cylinder part 22112 and has a first channel 22121 and a first opening 22122. The positioning part 2213 is located inside the upper cylinder part 22111 and is disposed outside the conduit 2212. The first snap-on part 2214 includes an arm part 22141, a connecting part 22142 and a hook part 22143. The connecting part 22142 is disposed at the upper cylinder part 22111, passes through the first through hole 22114 from the inside of the upper cylinder part 22111, and connects the arm part 22141; and the hook part 22143 is disposed at one end of the arm part 22141 and located in the second through hole 22115. The first coupling part 222 includes a first end part 2221, a second end part 2222 and a connecting channel 2223. The first end part 2221 is disposed at a second end of the upper cylinder part 22111 and has a groove 22211, and a groove wall of the groove 22211 is arc-shaped. The second end 2222 is an integral vial adaptor, has a puncturing member 1221, and is used to be combined with a vial bottle 100A (see FIGS. 1 and 9); and the connecting channel 2223 runs through the first end 2221 and the second end 2222 and communicates with the groove 22211. The first elastic valve 223 includes a first circumferential part 2231 and a first sealing part 2232. Two ends of the first circumferential part 2231 are respectively disposed at the positioning part 2213 and the first end part 2221. The first sealing part 2232 is located in the channel 22121. A first end of the first sealing part 2232 is connected to an inner side of the first circumferential part 2231 and disposed with a plurality of through holes 2233. The through holes 2233 are connected with the first channel 22121, the through holes 2233 are connected with the grooves 22211, and a second end of the first sealing part 2232 closes the first opening 22122.

The first needleless connector 23 includes a housing 231, a second coupling part 232, and a second elastic valve 233. The housing 231 includes an upper shell 2311, a lower shell 2312, a second snap-on part 2313, and a stabilizing structure 2314. A first end of the upper shell 2311 has a second opening 23111. An outer diameter of the upper shell 2311 is smaller than that of the lower shell 2311. The second snap-on part 2313 is a bump and is disposed on an outer side of a first end of the lower shell 2312. The stabilizing structure 2314 is a bump and is disposed on an outer side of a second end of the lower shell 2312. The second coupling part 232 includes a first end part 2321 and a second end part 2322. The first end part 2321 is disposed at a second end of the lower shell 2312. The second end part 2322 has a third opening 23222 and is used to combine with the coupling seat 121. The second elastic valve 233 includes a second circumferential part 2331 and a second sealing part 2332. The second circumferential part 2331 is disposed inside the lower shell 2312 and the second coupling part 232 and has a second channel 23311. The second channel 23311 communicates with the third opening 23222. The second sealing part 2332 is disposed inside the upper shell 2311 and has a slit 23321. The second sealing part 2332 closes the second opening 23111.

As shown in FIG. 9, and refer to FIG. 4, when the movable closed connector 22 is docked with the first needleless connector 23, the second snap-on part 2313 is located in the second through hole 22115, and the hook part 22143 is fixed on a bottom end of the second snap-on part 2313. The conduit 2212 compresses the second sealing part 2332, the second sealing part 2332 compresses the second circumferential part 2331, so that the second sealing part 2332 is away from the second opening 23111, and the conduit 2212 passes through the slit 23321 and enters the second sealing part 2332. The first end of the upper shell 2311 is against an inner wall of the middle cylinder part 22112. The positioning part 2213 moves along the conduit 2212 in a direction away from the first needleless connector 23 and compresses the first circumferential part 2231, so that the second end of the first sealing part 2232 is separated from the first opening 22122, and the first channel 22121 communicates with the second channel 23311. An inner side of the lower cylinder 22113 abuts an outer side of the stabilizing structure 2314, so the lower shell 2312 will not rock in the lower barrel 22113.

As shown in FIG. 4, and refer to FIG. 9, when the movable closed connector 22 is separated from the first needleless connector 23, the arm part 22141 is pressed so that the hook part 22143 is separated from the bottom end of the second snap-on part 2313. The conduit 2212 is separated from the second sealing part 2332, so that the slit 23321 is tightly closed. The second sealing part 2332 is reset by the elastic force of the second circumferential part 2331 and closes the second opening 23111. The first end of the upper shell 2311 is separated from the middle cylinder part 22112. The positioning part 2213 and the first sealing part 2232 are reset by the elastic force of the first circumferential part 2231, so that the second end of the first sealing part 2232 closes the first opening 22122.

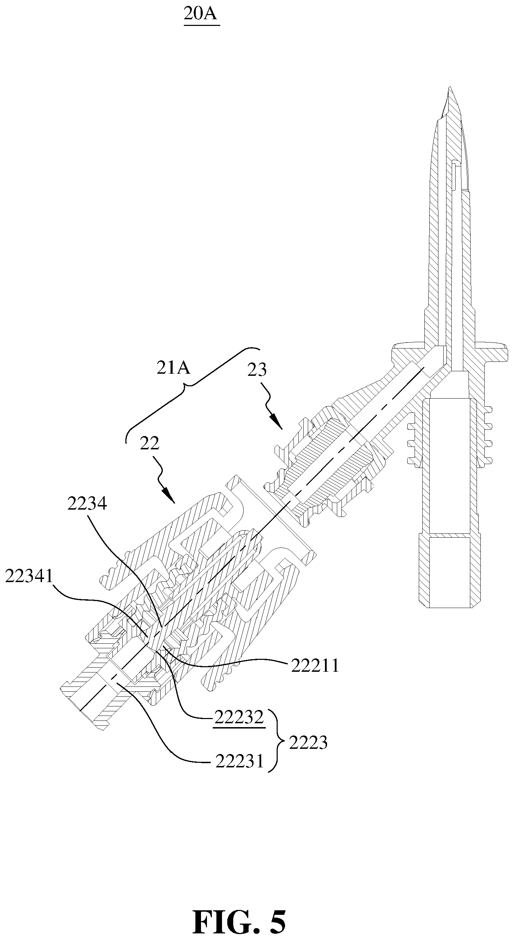

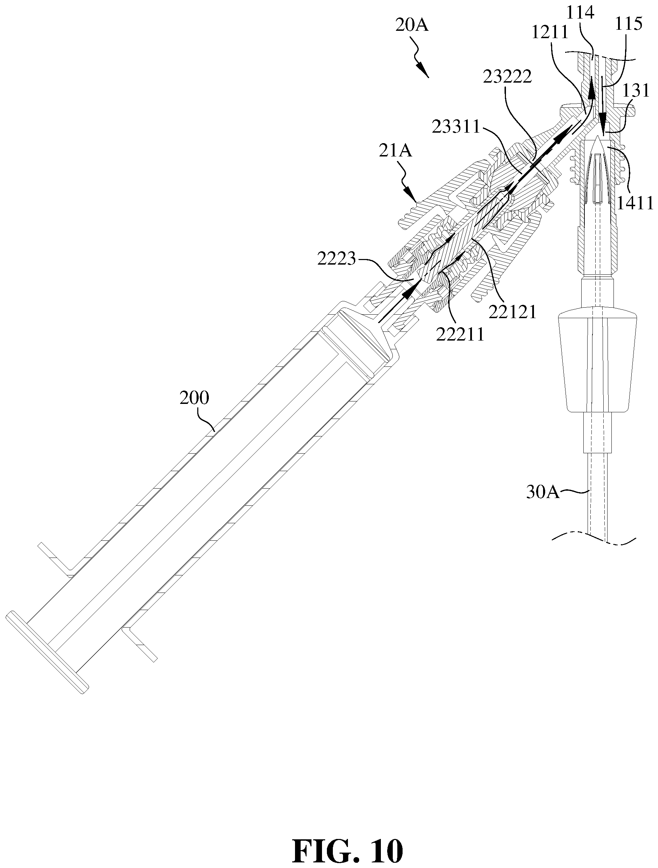

As shown in FIGS. 5 and 10, the first closed needleless connector module 21A differs from the first closed needleless connector module 21 in that: first, the second end 2222 is a Luer connector and is used to be combined with a syringe 200 (see FIGS. 1 and 10); second, the connecting channel 2223 includes a large-diameter part 22231 and a small-diameter part 22232; third, the first elastic valve 223 further includes a insertion rod 2234, a first end of the insertion rod 2234 is disposed at the first end of the first sealing part. A second end of the insertion rod 2234 has a head 22341. A diameter of the head 22341 is equal to a diameter of the small-diameter part 22232 and smaller than a diameter of the large-diameter part 22231. As shown in FIG. 5, when the movable closed connector 22 is docked with the first needleless connector 23, the head 22341 is located in the large-diameter part 22231 and forms a gap with a hole wall of the large-diameter part 22231, so that the connecting channel 2223 communicates with groove 22211. As shown in FIG. 5, when the movable closed connector 22 is separated from the first needleless connector 23, the head 22341 is located in the small-diameter part 22232 and abuts against an inner side of the small-diameter part 22232 to close the connecting channel 2223. Except for the above, the rest of the technical features of the second medicine mixing device 20A are exactly the same as those of the second medicine mixing device 20.

As shown in FIGS. 1 and 9, the puncturing part 11 of the second medicine mixing device 20A passes through the blocking part 1412 of the second medicine mixing device 20, so that the second medicine mixing device 20 and the second medicine mixing device 20A can be connected in series.

In some embodiments, a plurality of second medicine mixing devices 20, 20A can be connected in series with each other in the above-mentioned manner, or a plurality of second medicine mixing devices 20 can be connected in series in the above-mentioned manner, or a plurality of second medicine mixing devices 20A can be connected in series in the above-mentioned manner, which are all possible implementations. In some embodiments, the infusion system may also only include the second medicine mixing device 20 or the second medicine mixing device 20A.

As shown in FIGS. 1, 8 and 10, a first end of the infusion drip set 30 passes through the blocking part 1412 of the first mixing device 10, thereby allowing the infusion drip set 30 to communicate with the second flow channel 131 through the third flow channel 1411 of the first mixing device 10. A first end of the infusion drip set 30A passes through the blocking part 1412 of the second medicine mixing device 20A, thereby allowing the infusion drop set 30A to communicate with the second flow channel 131 through the third flow channel 1411 and the second medicine mixing device 20A.

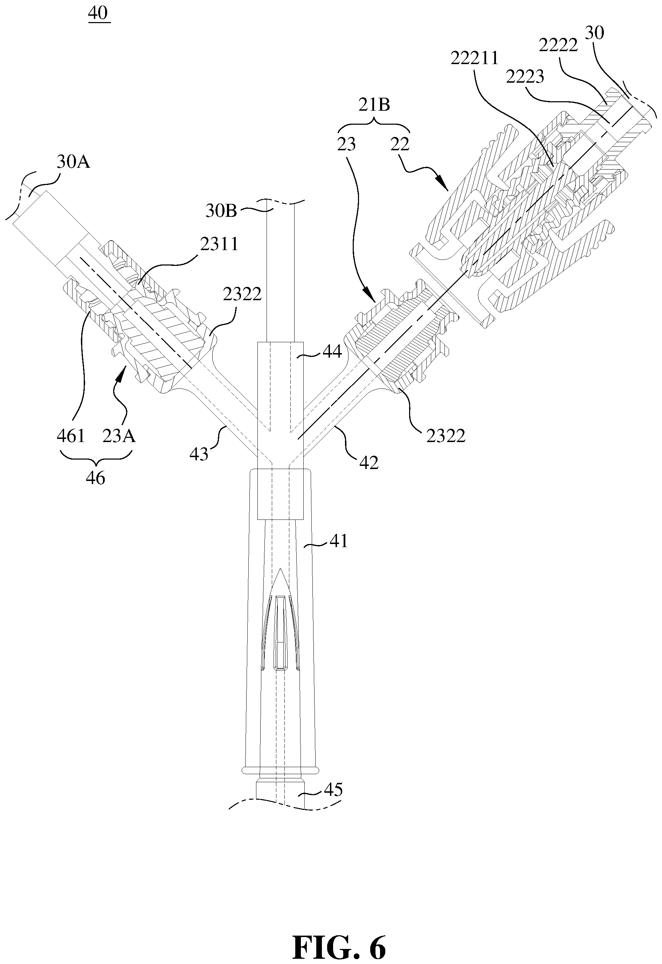

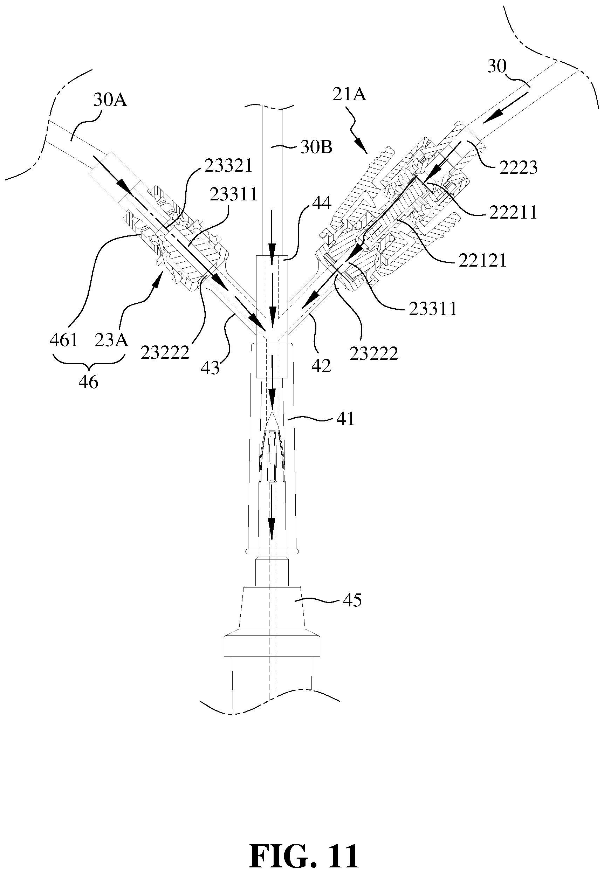

As shown in FIGS. 6 and 11, the dispenser 40 includes a gathering part 41, three input terminals 42, 43, 44, an output terminal 45, a second closed needleless connector module 21B, and a screw-coupling needleless connector module 46. The input terminals 42, 43 and 44 are connected to a first end of the gathering part 41. The output terminal 45 is connected to a second end of the gathering part 41. The second closed needleless connector module 21B differs from the first closed needleless connector module 21A in that: first, the first elastic valve 223 does not include the insertion rod 2234; second, the second end 2222 is connected to a second end of the infusion drip set 30; and third, the second end 2322 is coupled to the input terminal 42. The screw-coupling needleless connector module 46 includes a screw-coupling part 461 and a second needleless connector 23A. The screw-coupling part 461 is disposed at a second end of the infusion drip set 30A. The second needleless connector 23A has the same structure as that of the first needleless connector 23. The upper shell 2311 of the second needleless connector 23A is screwed to the screw-coupling part 461, and the second end 2322 of the second needleless connector 23A is disposed at the input terminal 43. The input terminal 44 is disposed at a second end of the infusion drip set 30B. The operation of the second closed needleless connector module 21B is almost the same as that of the first closed needleless connector module 21A. The only difference is that the connecting channel 2223 remains in communication with the groove 22211 and will not be closed.

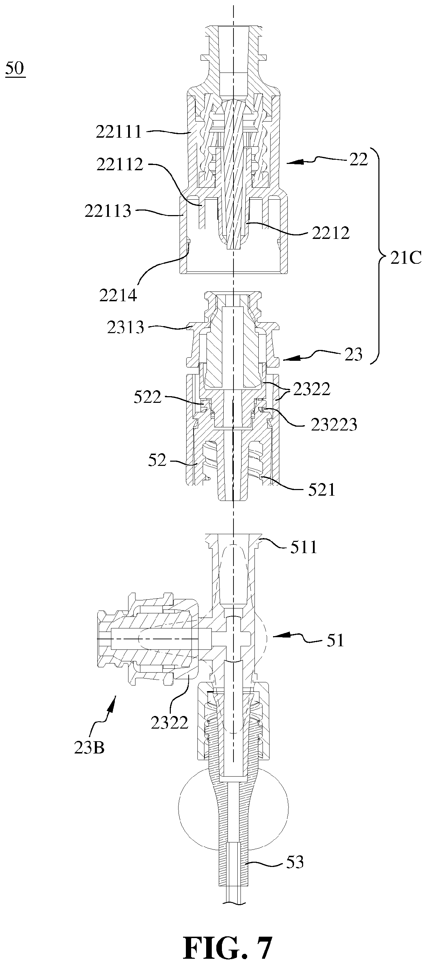

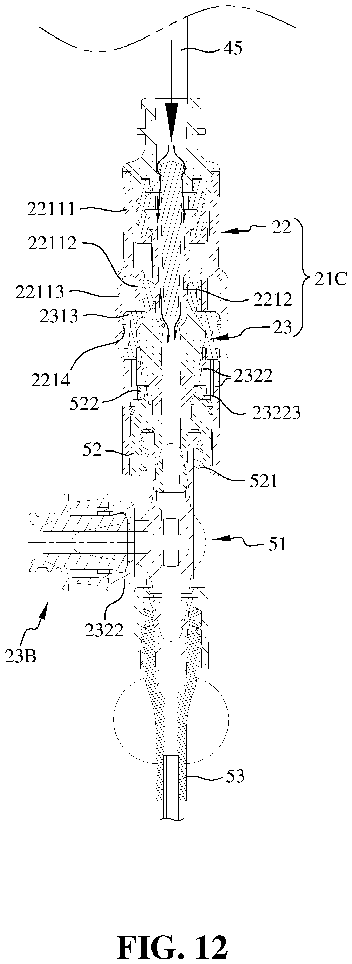

As shown in FIGS. 7 and 12, the separator 50 includes a valve body 51, a screw-coupling structure 52, an output terminal 53, a third needleless connector 23B and a third closed needleless connector module 21C. The valve body 51 is a three-way valve. The internal thread 521 of the screw-coupling structure 52 is threaded to the external thread 511 of a first end of the valve body 51. A first end of the output terminal 53 is disposed at a second end of the valve body 51, the second end part 2322 is disposed at a third end of the valve body 51. The third closed needleless connector module 21C differs from the first closed needleless connector module 21A in that: first, the middle cylinder part 22112 is disposed at an inner side of the first end of the upper cylinder part 22111, and the lower cylinder part 22113 is disposed on an outer side of the first end of the upper cylinder part 22111, the middle cylinder part 22112 extends inside the lower cylinder part 22113; second, the conduit 2212 extends from the inside of the upper cylinder part 22111 through the inside of the middle cylinder part 22112 to the lower cylinder part 22113; third, the first snap-on part 2214 is a bump, which is disposed on an inner side of the lower cylinder part 22113 and is located below the middle cylinder part 22112; fourth, the second end part 2232 is disposed on the screw-coupling structure 52, the outer surface of a first end of the screw-coupling structure 52 has a plurality of pawls 522, a second end of the screw-coupling structure 52 has an internal thread 521, the bottom end of the second end part 2322 is a ratchet with an inner side surface having a plurality of tooth parts 23223. The structure of the third needleless connector 23B is exactly the same as that of the first needleless connector 23. As shown in FIG. 7, when the movable closed connector 22 is docked with the first needleless connector 23, the first snap-on part 2214 is fixed on a bottom end of the second snap-on part 2313, and the force range of fixing the first snap-on part 2214 to the second snap-on part 2313 is between 1 and 6 pounds per foot. As shown in FIG. 12, when the movable closed connector 22 is separated from the first needleless connector 23, the first snap-on part 2214 is separated from the bottom end of the second snap-on part 2313. Except the above, the third closed needleless connector module 21C has the same technical features as the first closed needleless connector module 21 A.

As shown in FIG. 1, the retention needle 60 is disposed at a second end of the output terminal 53.

The following describes how the infusion system performs a medicine mixing procedure to evenly mix the high-concentration medicine liquid in the two vial bottles 100 and 100A and a syringe 200 and the diluting solution in the two infusion liquid bags 300 and 300A.

Step (1), according to the doctor's prescription, take out the vial bottles 100 and 100A containing a certain powder ingredient; according to the doctor's prescription, take out the infusion liquid bags 300 and 300A containing the diluting solution, the diluting solution can be saline or glucose solution; as shown in FIG. 8, the vial bottle 100 is sleeved on the integral vial adaptor 122 of the first medicine mixing device 10, and the puncturing member 1221 of the first medicine mixing device 10 puncture the cap 110 of the vial bottle 100; as shown in FIG. 9, the vial bottle 100A is sleeved on the second end 2222 of the closed needleless connector module 21, and the puncturing member 1221 of the closed needleless connector module 21 puncture the cap 110 of the vial bottle 100A; and as shown in FIG. 1, FIG. 8 and FIG. 9, a first end of the puncturing part 11 punctures a film of an infusion tube of the infusion liquid bags 300, 300A, and the infusion drip set 30B directly punctures a film of an infusion tube of the infusion liquid bag 300B.

Step (1-1), the infusion liquid bags 300, 300A are at the top, and the infusion system is at the bottom; the infusion liquid bag 300 is squeezed, and the diluting solution in the infusion liquid bag 300 passes through the U-shaped through hole 111 of the first mixing device 10, the first long hole 114, the first flow channel 1211, and the puncturing member 1221 to enter the inside of the vial bottle 100; the infusion liquid bag 300A is at the top, and the infusion system is at the bottom. Squeeze the infusion liquid bag 300A, and the diluting solution in the infusion liquid bag 300A passes through the U-shaped through hole 111, the first long hole 114, the first flow channel 1211, the third opening 23222, the second channel 23311, the first channel 22121, the through holes 2233, the groove 22211, and the connecting channel 2223 and the puncturing member 1221 of the second chemical mixing device 20 to enter the inside of the vial bottle 100A; and shakes the vial bottles 100 and 100A to slightly mix the powder and diluting solution inside the vial bottles 100 and 100A to obtain a high-concentration medicinal liquid.

Step (2), the infusion liquid bags 300, 300A are at the bottom, and the infusion system is at the top; the infusion liquid bag 300 is squeezed, and the air in the infusion liquid bag 300 passes through the U-shaped through holes 111, the first long hole 114, the first flow channel 1211 and the puncturing member 1221 of the first mixing device 10 in sequence to enter the inside of the vial bottle 100; and the infusion liquid bag 300A is squeezed, the air in the infusion liquid bag 300A passes through the U-shaped through hole 111, the first long hole 114, the first flow channel 1211, the third opening 23222, the second channel 23311, the first channel 22121, the through holes 2233, the groove 22211, the connecting channel 2223 and the puncturing member 1221 of the second medicine mixing device 20 to enter the inside of the vial bottle 100A.

Step (3), as shown in FIG. 8, loosen the infusion liquid bag 300, and the high-concentration medicinal liquid in the vial bottle 100 passes through the puncturing member 1221, the first flow channel 1211, the long hole 114 and the U-shaped through hole 111 of the first medicine mixing device 10 in sequence to enter the inside of the infusion liquid bag 300; as shown in FIG. 9, loosen the infusion liquid bag 300A, and the high-concentration medicinal liquid in the vial bottle 100A passes through the puncturing member 1221, the connecting channel 2223, the groove 22211, the through holes 2233, the first channel 22121, the second channel 23311, the third opening 23222, the first flow channel 1211, the first long hole 114 and the U-shaped through hole 111 of the second medicine mixing device 20 in sequence to enter the inside of the infusion liquid bag 300A; as shown in FIG. 10, the high-concentration medical liquid in the syringe 200 passes through the connecting channel 2223, the groove 22211, the through holes 2233, the first channel 22121, the second channel 23311, the third opening 23222, the first flow channel 1211, the first elongated hole 114 and the U-shaped through hole 111, the third flow channel 1411, the second flow channel 131, the two long holes 115 and the side holes 112 of the second medicine mixing device 20A in sequence to enter the infusion liquid bag 300A.

By repeatedly performing steps (2) and (3), all the high-concentration medicinal liquids in the vial bottles 100, 100A and syringes 200 can enter the inside of the infusion liquid bags 300, 300A, and at the same time, the high-concentration medicine solution in the infusion liquid bags 300 and 300A and the diluting solution are thoroughly mixed to obtain the diluted medicine.

It is worth noting that during the execution of the medicine mixing procedure, because the distance D1 from the U-shaped through hole 111 to the sharp end of the puncturing part 11 is smaller than the distance D2 from the side hole 112 to the sharp end of the puncturing part 11, and at the same time, the larger and longer the diameter of the puncturing part 11 is, the gap in the infusion tube of the infusion liquid bag 300, 300A of the puncturing of the puncturing part 11 is smaller, the flow space is smaller, and a tension is generated to block the medical solution, thereby preventing high-concentration medicinal liquid from entering the side holes 112.

The following will describe how the infusion system performs the infusion procedure, thereby infusing the diluted medicinal liquid into the patient's body.

Step (a), as shown in FIG. 1, the infusion liquid bags 300, 300A, 300B are at the top, and the infusion system is at the bottom; as shown in FIGS. 8 and 11, the diluted medicine in the infusion liquid bag 300 passes through the side holes 112, the second long holes 115, the second flow channel 131, the third flow channel 1411, the infusion drip set 30 of the medicine mixing device 10 and the connecting channels 2223, the groove 22211, the through holes 2233, the first channel 22121, the second channel 23311, the third opening 23222 and the input terminal 42 of the closed needleless connector module 21A to enter the inside of the gathering part 41; as shown in FIGS. 9, 10 and 11, the diluted medicine in the infusion liquid bag 300A passes through the side holes 112, the second long holes 115, the second flow channel 131, the third flow channel 1411 of the second medicine mixing device 20, the side holes 112, the second elongated holes 115, the second flow channel 131, the third flow channel 1411, the infusion drip sleeve 30A of the second medicine mixing device 20A, the screw-coupling part 461 of the screw-coupling needless connector module 46, the slit 23321, the second channel 23311, the third opening 23222 and the input terminal 43 of the needleless connector to enter the inside of the gathering part 41; and as shown in FIGS. 1 and 11, other medicines in the infusion liquid bag 300B pass through the infusion drip set 30B and the input terminal 44 in sequence to enter the inside of the gathering part 41.

Step (b), as shown in FIGS. 11 and 12, after the three medicines are mixed inside the gathering part 41, the mixed medicine passes through the output terminal 45 and then enter the third closed needleless connector module 21C.

Step (c), as shown in FIGS. 1 and 12, the mixed medicine passes through the connecting channel 2223, the groove 22211, the through holes 2233, and the first channel 22121, the second channel 23311, the third opening 23222 of the closed needleless connector module 21C, the screw-coupling structure 52, the valve body 51, the output end 53 of the separator 50, and the retention needle 60 in sequence to enter the patient's body.

Thus, because the U-shaped through hole 111 and the side holes 112 are separated by the first partition 113, and the first flow channel 1211 and the second flow channel 131 are separated by the second partition 15, in step (3), the high-concentration medicinal solution in the vial bottle 100 will only pass through the puncturing member 1221, the first flow channel 1211, the first long hole 114 of the puncturing part 11, and the U-shaped through hole 111 of the first medicine mixing device 10 in sequence, instead of passing through the second flow channel 131, the second long holes 115 and the side holes 112 of the first medicine mixing device 10 in sequence; and the high-concentration medicinal liquid in the vial bottle 100A will only pass through the puncturing member 1221, the connecting channel 2223, the groove 22211, the through holes 2233, the first channel 22121, the second channel 23311, the third opening 23222, the first flow channel 1211, the first long hole 114 and the U-shaped through hole 111 of the second chemical mixing device 20, instead of passing through the second flow channel 131, the second elongated holes 115 and the side holes 112 of the second chemical mixing device 20 in sequence. Therefore, when the high-concentration medicinal liquid in the vial bottles 100 and 100A passes through the U-shaped through hole 111 of the first medicine mixing device 10 and the second medicine mixing device 20, there will not be any high-concentration medicinal liquid attached to the inner side walls of the side holes 112.

When step (2) is performed again, since there is no high-concentration medicinal liquid attached to the inner walls of the side holes 112, even if the air in the infusion liquid bags 300 and 300A passes through the side holes 112, there will not be any high-concentration liquid medicine remaining in the space between the second flow channel 131 and insertion hole 132.

Since there is no high-concentration liquid medicine remaining in the space between the second flow channel 131 and the insertion hole 132, during the infusion process, only the diluted medicine can pass through the side holes 112, the second long hole 115, the second flow channel 131, the third flow channel 1411, the infusion drip set 30, 30A, the dispenser 40, the separator 50, and the retention needle 60 in sequence to enter the patient's body, the problem of residual high-concentration medicinal liquid entering the patient's body will not occur; thereby, the patient will not feel uncomfortable, and it is very safe and reliable.

Furthermore, because there is no high-concentration medical liquid remaining in the space between the second flow channel 131 and the insertion hole 132, the nursing staff does not need to discharge any residual high-concentration medicinal liquid, so that the infusion can achieve the prescribed dosage of medicine in the patient's body without any reduction and is consistent with the doctor's prescription, thereby maintaining the therapeutic effect and causing no medicine waste.

In addition, the first long hole 114 only allows the high-concentration medicinal liquid in the vial bottles 100 and 100A to pass into the infusion liquid bags 300 and 300A. The second long holes 115 only allow the diluted medicine in the infusion liquid bags 300 and 300A into the patient's body so as to prevent the diluted medicine from flowing back into the vial bottles 100 and 100A. As such, the dose infused into the patient's body is not reduced and is consistent with the doctor's prescription, thereby maintaining the therapeutic effect without causing any medicine waste.

Moreover, regarding the first closed needleless connector modules 21, 21A, when the movable closed connector 22 and the first needleless connector 23 are separated, both the movable closed connector 22 and the first needleless connector 23 have a sealing effect and can prevent liquid leakage. Therefore, the vial bottle 100A and the syringe 200 can be removed from the movable closed connector 22 so that the second medicine mixing device 20, 20A can be reused. If medical staff want to mix medicine a plurality of times, the same second medicine mixing device 20, 20A can be reused to install a plurality of vial bottles 100A or a plurality of syringes 200 in batches to mix medicine a plurality of times without replacing the second medicine mixing device 20 and 20A, and thereby the cost is saved. In practice, medical staff can also connect a plurality of second medicine mixing devices 20 and 20A in series, install a plurality of vial bottles 100A or a plurality of syringes 200 at one time, and complete the above-mentioned plurality of medicine mixing procedures at once, and thereby saving time. It should be noted that each time before installing the vial bottle 100A or the syringe 200, the movable closed connector 22 and the first needleless connector 23 must be sterilized.

In addition, regarding the second closed needleless connector module 21B, when the movable closed connector 22 is separated from the first needleless connector 23, both the movable closed connector 22 and the first needleless connector 23 have the sealing effect to prevent liquid leakage.

Moreover, regarding the third closed needleless connector module 21C, the force range of fixing the first snap-on part 2214 to the second snap-on part 2313 is between 1 and 6 pounds per foot, which allows easy separation. Even if the patient moves erratically, the movable closed connector 22 and the first needleless connector 23 can be immediately separated to prevent leakage of medical solution and blood.

It is worth noting that since the groove wall of the groove 22211 is arc-shaped, the medical liquid can flow along the arc-shaped groove wall of the groove 2221 to prevent the generation of eddy currents and thereby increase the flow rate of the medical liquid.

It is also worth noting that because the diameter of the groove 22211 is larger than the diameter of the first sealing part 2232, even if the first circumferential part 2231 is compressed and deformed and the first sealing part 2232 is close to the groove 22211, the groove 22211 remains open to these through holes 2233 and will not be affected.

In addition, the upper shell 2311 of the third needleless connector 23B is a Luer connector and is used to combine with a syringe 200. Thereby, the medical staff can take out the blood sample from or add non-toxic general medicine to the second end of the valve body 51 through the needleless connector 23 of the separator 50.

In some embodiments, the valve body 51 may also be a female Luer connector.

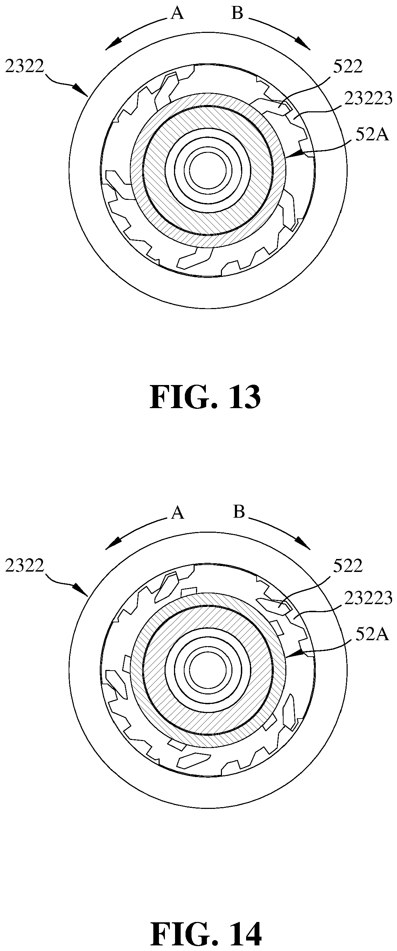

As shown in FIG. 13, and refer to FIG. 7, when the first needleless connector 23 rotates along a first direction A by a first rotation force, the first needleless connector 23 will transmit the first rotation force sequentially through the teeth 23223 and the pawls 522 to the screw-coupling structure 52. The screw-coupling structure 52 is pushed by the first rotation force and rotates along the first direction A, so that the screw-coupling structure 52 is fixed on the first end of the valve body 51. After the screw-coupling structure 52 is fixed on the first end of the valve body 51, when the first needleless connector 23 rotates along a second direction B opposite to the first direction A by a second rotation force, the first needleless connector 23 uses the second rotation force to squeeze the pawls 522 through the teeth part 23223, causing the pawls 522 to bend and deform to break away from the teeth part 23223, and the first needleless connector 23 rotates idlingly with respect to the screw-coupling structure 52 along the second direction B. At this time, the screw-coupling structure 52 is still fixed to the first end of the valve body 51, and the first needleless connector 23 is completely unable to drive the screw-coupling structure 52 to rotate in the second direction B.

As shown in FIG. 14, and refer to FIG. 7, after the screw-coupling structure 52 is fixed on the first end of the valve body 51, when the first needleless connector 23 rotates in direction A around the first end of the valve body 51 by a third rotation force, the first needleless connector 23 transmits the third rotation force to the pawls 522 through the tooth parts 23223. The third rotation force is greater than the force that the pawls 522 can withstand, and the pawls 522 are broken, so that the screw-coupling structure 52 can rotate arbitrarily along the first direction A and the second direction B with respect to the first needleless connector 23, so that the liquid medicine in the output terminal 53 can maintain smooth flow.

Although the present invention has been described with reference to the preferred embodiments thereof, it is apparent to those skilled in the art that a variety of modifications and changes may be made without departing from the scope of the present invention which is intended to be defined by the appended claims.

## Claims

1. A snap-on closed needleless connector module for infusion system, comprising:
a movable closed connector comprising: a body, a first coupling part, and a first elastic valve; the body further comprising: a sleeve, a conduit, a positioning part and a first snap-on part; the conduit being arranged inside the sleeve, and having a first channel and a first opening; the positioning part being located inside the sleeve and disposed on the outside of the conduit; the first snap-on part being disposed on the sleeve, and the first coupling part being disposed on the sleeve and used for coupling a vial bottle, a syringe, or an infusion drip set of an infusion system; the first elastic valve comprising: a first circumferential part and a first sealing part, two ends of the first circumferential part being respectively arranged on the positioning part and the first coupling part, the first sealing part being located in the first channel, a first end of the first sealing part being connected to an inner side of the first circumferential part and disposed with a plurality of through holes, and the through holes communicating with the first channel, and a second end of the first sealing part closing the first opening; and
a first needleless connector, comprising: a housing, a second coupling part, and a second elastic valve; the housing being disposed with a second snap-on part, and the second snap-on part being disposed on an outer side of the housing; a first end of the housing having a second opening, a second coupling part being disposed at a second end of the housing, having a third opening, and being used for coupling a coupling seat of a medicine mixing device of the infusion system, an input end of a dispenser of the infusion system, or an input end of a separator of the infusion system; the second elastic valve comprising: a second circumferential part and a second sealing part, the second circumferential part being disposed inside the housing and the second coupling part and has a second channel, the second channel communicating with the third opening; the second sealing part being disposed inside the housing and having a slit, and the second sealing part closing the second opening;
wherein, when the movable closed connector is docked with the first needleless connector, the first snap-on part is fixed on the second snap-on part, the conduit compresses the second sealing part, and the second sealing part compresses the second circumferential part, so that the second sealing part is away from the second opening, and the conduit passes through the slit of the second sealing part to enter the inside of the second sealing part, the first end of the housing is against an inner wall of the sleeve, and the positioning part moves along conduit in a direction away from the first needleless connector and compresses the first circumferential part, so that the second end of the first sealing part is separated from the first opening, and the first channel communicates with the second channel; and
wherein, when the movable closed connector is separated from the first needleless connector, the first snap-on part is separated from the second snap-on part, and the conduit is separated from the second sealing part, so that the slit of the second sealing part is tightly closed, the second sealing part is reset by the elastic force of the second circumferential part and closes the second opening; the first end of the housing is separated from the sleeve, the positioning part and the first sealing part are reset by the elastic force of the first circumferential part, so that the second sealing part is reset by the elastic force of the first circumferential part so that the second end of the first sealing part closes the first opening .

2. The snap-on closed needleless connector module according to claim 1, wherein the sleeve comprises an upper cylinder part, a middle cylinder part and a lower cylinder part, the middle cylinder part is disposed between a first end of the upper cylinder part and a first end of the lower cylinder part; a first through hole is disposed between a side wall of the upper cylinder part and a side wall of the middle cylinder part, and a second through hole is disposed between the side wall of the middle cylinder part and a side wall of the lower cylinder part; wherein the conduit extends from the inside of the upper cylinder part to the inside of the middle cylinder part; wherein the positioning part is located inside the upper cylinder part; wherein the first snap-on part comprises an arm part, a connecting part, and a hook part; the connecting part is disposed on the upper cylinder part, passes through the first through hole from the inside of the upper cylinder part, and is connected to the arm part; the hook part is disposed at one end of the arm part and is located in the second through hole; wherein, the first snap-on part is disposed at a second end of the upper cylinder part; wherein, the housing comprises an upper shell and a lower shell, a first end of the upper shell has the second opening, an outer diameter of the upper shell is smaller than an outer diameter of the lower shell, and the second snap-on part is a bump and is disposed on an outer side of a first end of the lower shell; wherein, when the movable closed connector is docked with the first needleless connector, the second snap-on part is located in the second through hole, and the hook part is fixed to a bottom end of the second snap-on part, the first end of the upper shell is against an inner wall of the middle cylinder part; wherein, when the movable closed connector is separated from the first needleless connector, the arm part is pressed so that the hook part is separated from the bottom end of the second snap-on part, and the first end of the upper shell is separated from the middle cylinder part.

3. The snap-on closed needleless connector module according to claim 1, wherein the sleeve comprises an upper cylinder part, a middle cylinder part and a lower cylinder part, and the middle cylinder part is disposed at an inner side of a first end of the upper cylinder part, the lower cylinder part is disposed at an outer side of the first end of the upper tube, so that the middle cylinder part extends inside the lower cylinder part; wherein the conduit extends from the inside of the upper cylinder part through the middle cylinder part to the inside of the lower cylinder part; wherein, the positioning part is located inside the upper cylinder part; wherein the first snap-on part is a bump, disposed on an inner side of the lower cylinder part and located below the middle cylinder part; wherein, the first snap-on part is disposed at a second end of the upper cylinder part; wherein, the housing comprises an upper shell and a lower shell, and a first end of the upper shell has the second opening, an outer diameter of the upper shell is smaller than an outer diameter of the lower shell, and the second snap-on part is a bump and is disposed on an outer side of a first end of the lower shell; wherein, when the movable closed connector is docked with the first needleless connector, the first snap-on part is fixed on a bottom end of the second snap-on part, and a first end of the upper shell abuts against an inner wall of the middle cylinder part.

4. The snap-on closed needleless connector module according to claim 3, wherein the force range of fixing the first snap-on part to the second snap-on part is between 1 and 6 pounds per foot.

5. The snap-on closed needleless connector module according to claim 1, wherein the first coupling part comprises a first end part, a second end part and a connecting channel, and the first end of the first coupling part is provided on the sleeve and has a groove having an arc-shaped groove wall; the second end of the first coupling part is used to couple to the vial bottle, the syringe, or the infusion drip set of the infusion system, the connecting channel runs through the first end and the second end of the first coupling part and communicates with the groove, and the groove communicates with the through holes; wherein the two ends of the first elastic valve are respectively disposed on the positioning part and the first end of the first coupling part.

6. An infusion system, comprising:
a first mixing device, comprising: a puncturing part, a needleless additive port, an output part, and a plug member; a first end of the puncturing part being a sharp end, and a U-shaped through hole and at least one side hole being disposed on one side wall of the puncturing part, a distance from the top of the U-shaped through hole to the sharp end of the puncturing part being less than a distance from the top of the at least one side hole to the sharp end of the puncturing part, and the puncturing part being divided by a first partition to divide the internal space into a first long hole and at least a second long hole; the first long hole communicating with the U-shaped through hole, and the at least one second long hole communicates with the at least one side hole; the needleless additive port comprising a coupling seat and an integral vial adaptor, the coupling seat being integrally formed on one side of the second end of the puncturing part, and disposed with a first flow channel; the first flow channel and the U-shaped through hole communicating with each other, the integral vial adaptor being integrally formed on the coupling seat, having a puncturing member, and being used for engaging with a vial bottle; the output part being disposed at the second end of the puncturing part, and being disposed with a second flow channel and an insertion hole; the second flow channel being connected between the at least one second long hole and the insertion hole, wherein a second partition being used between the needleless additive port and the output part to separate the first flow channel and the second flow channel, and the first partition being connected to the second partition; the plug member having a tube body, the tube body defining a third flow channel, and a first end of the tube body being inserted into insertion hole, so that the second flow channel communicating with the third flow channel, and a blocking part being disposed inside the third flow channel to close the third flow channel;
at least one second medicine mixing device comprising: a puncturing part, a needleless additive port, an output part, a plug member, and a snap-on closed needleless connector module as claimed in claim 1, and a first end of the puncturing part being a sharp end; a U-shaped through hole and at least one side hole being disposed on one side wall of the puncturing part; a distance from the top of the U-shaped through hole to the sharp end of the puncturing part being less than a distance from the top of the at least one side hole to the sharp end of the puncturing part; the puncturing part being divided divides by a first partition to dividing internal space into a first long hole and at least a second long hole ; the first long hole and the U-shaped through holes communicating with each other, the at least one second long hole communicating with the at least one side hole; the needleless additive port comprising a coupling seat, the coupling seat being integrally formed on one side of the second end of the puncturing part and being disposed with a first flow channel, and the first flow channel communicating with the U-shaped through hole; the output part being disposed at the second end of the puncturing part and being disposed with a second flow channel and an insertion hole, the second flow channel communicating between the at least one second long hole and the insertion hole, wherein a second partition being disposed between the needleless additive port and the output part to separate the first flow channel and the second flow channel, and the first partition being connected to the second partition, the plug member having a tube body, the tube body defining a third flow channel, a first end of the tube body being inserted into the insertion hole, so that the second flow channel and the third flow channel channels communicating with each other, and a blocking part being disposed inside the third flow channel to close the third flow channel; the closed needleless connector module as mentioned above being defined as a first closed needleless connector module, and the first coupling part of the first closed needleless connector module being used for coupling to a vial bottle or a syringe, and the second coupling part of the first closed needleless connector module being used for coupling to the coupling seat;
a first end of one of the at least two infusion drip sets passing through the blocking part of the first mixing device, thereby allowing one of the infusion drip sets to communicate with the second flow channel through the third flow channel of the first medicine mixing device, and a first end of the other one of the infusion drip sets passing through the blocking part of the at least one second medicine mixing device, thereby allowing the other one of the infusion drip sets to communicate with the second flow channel through the third flow channel of the at least one second medicine mixing device;
a dispenser, comprising: a gathering part, at least two input terminals, an output terminal, a closed needleless connector module as claimed in claim 1, and a screw-type needleless connector module; the input terminals being connected to a first end of the gathering part, the output terminal being connected to a second end of the gathering part, the closed needleless connector module being defined as a second closed needleless connector module, the first coupling part of the second closed needleless connector module being used for coupling to a second end of one of the infusion drip sets, and the second coupling part of the second closed needleless connector module being used for coupling to one of the input terminals; the screw-type needleless connector module comprising a screw-coupling part and a second needleless connector; the screw-coupling part being disposed on the second end of sleeve of the other one of the infusion drip sets, a first end of the second needleless connector being screwed to the screw-coupling part, and a second end of the second needleless connector being disposed on the other one of the input terminals;
a separator, comprising: a valve body, a screw-coupling structure, an output terminal, a third needleless connector, and a closed needleless connector module as claimed in claim 1; an internal thread of a first end of the screw-coupling structure being screwed to an external thread of a first end of the valve body and defined as an input terminal of the separator; a first end of the output terminal being disposed on a second end of the valve body; the third needleless connector being disposed at a third end of the valve body; the closed needleless connector module being defined as a third closed needleless connector module, and the second coupling part of the third closed needleless connector module being used for coupling to the screw-coupling structure; and
a retention needle, being disposed at a second end of the output terminal of the separator.

## Amended claims

### Amended claims in accordance with Rule 137(2) EPC.

1. A snap-on closed needleless connector module (21, 21A, 21B, 21C) for infusion system, comprising:
a movable closed connector (22) comprising: a body (221), a first coupling part (222), and a first elastic valve (223); the body (221) further comprising: a sleeve (2211), a conduit (2212), a positioning part (2213) and a first snap-on part (2214); the conduit (2212) being arranged inside the sleeve (2211), and having a first channel (22121) and a first opening (22122); the positioning part (2213) being located inside the sleeve (2211) and disposed on an outside of the conduit (2212); the first snap-on part (2214) being disposed on the sleeve (2211), and the first coupling part (222) being disposed on the sleeve (2211) and used for coupling a vial bottle (100A), a syringe (200), or an infusion drip set (30A) of an infusion system; the first elastic valve (223) comprising: a first circumferential part (2231) and a first sealing part (2232), two ends of the first circumferential part (2231) being respectively arranged on the positioning part (2213) and the first coupling part (222), the first sealing part (2232) being located in the first channel (22121), a first end of the first sealing part (2232) being connected to an inner side of the first circumferential part (2231) and disposed with a plurality of through holes (2233), and the through holes (2233) communicating with the first channel (22121), and a second end of the first sealing part (2232) closing the first opening (22122); and
a first needleless connector (23), comprising: a housing (231), a second coupling part (232), and a second elastic valve (233); the housing (231) being disposed with a second snap-on part (2313), and the second snap-on part (2313) being disposed on an outer side of the housing (231); a first end of the housing (231) having a second opening (23111), a second coupling part (232) being disposed at a second end of the housing (231), having a third opening (23222), and being used for coupling a coupling seat (121) of a medicine mixing device (10, 20, 20A) of the infusion system, an input terminal (42) of a dispenser (40) of the infusion system, or an input terminal (52) of a separator (50) of the infusion system; the second elastic valve (233) comprising: a second circumferential part (2331) and a second sealing part (2332), the second circumferential part (2331) being disposed inside the housing (231) and the second coupling part (232) and has a second channel (23311), the second channel (23311) communicating with the third opening (23222); the second sealing part (2332) being disposed inside the housing (231) and having a slit (23321), and the second sealing part (2332) closing the second opening (23111);
wherein, when the movable closed connector (22) is docked with the first needleless connector (23), the first snap-on part (2214) is fixed on the second snap-on part (2313), the conduit (2212) compresses the second sealing part (2332), and the second sealing part (2332) compresses the second circumferential part (2331), so that the second sealing part (2332) is away from the second opening (23111), and the conduit (2212) passes through the slit (23321) of the second sealing part (2332) to enter the inside of the second sealing part (2332), the first end of the housing (231) is against an inner wall of the sleeve (2211), and the positioning part (2213) moves along conduit (2212) in a direction away from the first needleless connector (23) and compresses the first circumferential part (2231), so that the second end of the first sealing part (2232) is separated from the first opening (22122), and the first channel (22121) communicates with the second channel (23311); and
wherein, when the movable closed connector (22) is separated from the first needleless connector (23), the first snap-on part (2214) is separated from the second snap-on part (2313), and the conduit (2212) is separated from the second sealing part (2332), so that the slit (23321) of the second sealing part (23321) is tightly closed, the second sealing part (2332) is reset by an elastic force of the second circumferential part (2331) and closes the second opening (23111); the first end of the housing (231) is separated from the sleeve (2211), the positioning part (2213) and the first sealing part (2232) are reset by an elastic force of the first circumferential part (2231), so that the second end of the first sealing part (2232) closes the first opening (22122).

2. The snap-on closed needleless connector module (21, 21A, 21B, 21C) according to claim 1, wherein the sleeve (2211) comprises an upper cylinder part (22111), a middle cylinder part (22112) and a lower cylinder part (22113), the middle cylinder part (22112) is disposed between a first end of the upper cylinder part (22111) and a first end of the lower cylinder part (22113); a first through hole (22114) is disposed between a side wall of the upper cylinder part (22111) and a side wall of the middle cylinder part (22112), and a second through hole (22115) is disposed between the side wall of the middle cylinder part (22112) and a side wall of the lower cylinder part (22113); wherein the conduit (2212) extends from an inside of the upper cylinder part (22111) to an inside of the middle cylinder part (22112); wherein the positioning part (2213) is located inside the upper cylinder part (22111); wherein the first snap-on part (2214) comprises an arm part (22141), a connecting part (22142), and a hook part (22143); the connecting part (22142) is disposed on the upper cylinder part (22111), passes through the first through hole (22114) from the inside of the upper cylinder part (22111), and is connected to the arm part (22141); the hook part (22143) is disposed at one end of the arm part (22141) and is located in the second through hole (22115); wherein, the first snap-on part (2214) is disposed at a second end of the upper cylinder part (22111); wherein, the housing (231) comprises an upper shell (2311) and a lower shell (2312), a first end of the upper shell (2311) has the second opening (23111), an outer diameter of the upper shell (2311) is smaller than an outer diameter of the lower shell (2312), and the second snap-on part (2313) is a bump and is disposed on an outer side of a first end of the lower shell (2312); wherein, when the movable closed connector (22) is docked with the first needleless connector (23), the second snap-on part (2313) is located in the second through hole (22115), and the hook part (22143) is fixed to a bottom end of the second snap-on part (2313), the first end of the upper shell (2311) is against an inner wall of the middle cylinder part (22112); wherein, when the movable closed connector (22) is separated from the first needleless connector (23), the arm part (22141) is pressed so that the hook part (22143) is separated from the bottom end of the second snap-on part (2313), and the first end of the upper shell (2311) is separated from the middle cylinder part (22112).

3. The snap-on closed needleless connector module (21, 21A, 21B, 21C) according to claim 1, wherein the sleeve (2211) comprises an upper cylinder part (22111), a middle cylinder part (22112) and a lower cylinder part (22113), and the middle cylinder part (22112) is disposed at an inner side of a first end of the upper cylinder part (22111), the lower cylinder part (22113) is disposed at an outer side of the first end of the upper cylinder part (22111), so that the middle cylinder part (22112) extends inside the lower cylinder part (22113); wherein the conduit (2212) extends from an inside of the upper cylinder part (22111) through the middle cylinder part (22112) to the inside of the lower cylinder part (22113); wherein, the positioning part (2213) is located inside the upper cylinder part (22111); wherein the first snap-on part (2214) is a bump, disposed on an inner side of the lower cylinder part (22113) and located below the middle cylinder part (22112); wherein, the first snap-on part (2214) is disposed at a second end of the upper cylinder part (22111); wherein, the housing (231) comprises an upper shell (2311) and a lower shell (2312), and a first end of the upper shell (2311) has the second opening (23111), an outer diameter of the upper shell (2311) is smaller than an outer diameter of the lower shell (2312), and the second snap-on part (2313) is a bump and is disposed on an outer side of a first end of the lower shell (2312); wherein, when the movable closed connector (22) is docked with the first needleless connector (23), the first snap-on part (2214) is fixed on a bottom end of the second snap-on part (2313), and a first end of the upper shell (2311) abuts against an inner wall of the middle cylinder part (22112).

4. The snap-on closed needleless connector module (21, 21A, 21B, 21C) according to claim 3, wherein the force range of fixing the first snap-on part (2214) to the second snap-on part (2313) is between 1.4 and 8.1 Newton metres (1 and 6 pounds per foot).

5. The snap-on closed needleless connector module (21, 21A, 21B, 21C) according to claim 1, wherein the first coupling part (222) comprises a first end part (2221), a second end part (2222) and a connecting channel (2223), and the first end part (2221) of the first coupling part (222) is provided on the sleeve (2211) and has a groove (22211) having an arc-shaped groove wall; the second end part (2222) of the first coupling part (222) is used to couple to the vial bottle (100A), the syringe (200), or the infusion drip set (30A) of the infusion system, the connecting channel (2223) runs through the first end part (2221) and the second end part (2222) of the first coupling part (222) and communicates with the groove (22211), and the groove (22211) communicates with the through holes (2233); wherein the two ends of the first elastic valve (223) are respectively disposed on the positioning part (2213) and the first end of the first coupling part (222).

6. An infusion system, comprising:
a first medicine mixing device (10), comprising: a puncturing part (11), a needleless additive port (12), an output part (13), and a plug member (14); a first end of the puncturing part (11) being a sharp end, and a U-shaped through hole (111) and at least one side hole (112) being disposed on one side wall of the puncturing part (11), a distance (D1) from the top of the U-shaped through hole (111) to the sharp end of the puncturing part (11) being less than a distance (D2) from the top of the at least one side hole (112) to the sharp end of the puncturing part (11), and the puncturing part (11) being divided by a first partition (113) to divide an internal space into a first long hole (114) and at least a second long hole (115); the first long hole (114) communicating with the U-shaped through hole (111), and the at least one second long hole (115) communicates with the at least one side hole (112); the needleless additive port (12) comprising a coupling seat (121) and an integral vial adaptor (122), the coupling seat (121) being integrally formed on one side of a second end of the puncturing part (11), and being disposed with a first flow channel (1211); the first flow channel (1211) and the U-shaped through hole (111) communicating with each other, the integral vial adaptor (122) being integrally formed on the coupling seat (121), having a puncturing member (1221), and being used for engaging with a vial bottle (100); the output part (13) being disposed at the second end of the puncturing part (11), and being disposed with a second flow channel (131) and an insertion hole (132); the second flow channel (131) being connected between the at least one second long hole (115) and the insertion hole (132), wherein a second partition (15) being used between the needleless additive port (12) and the output part (13) to separate the first flow channel (1211) and the second flow channel (132), and the first partition (113) being connected to the second partition (15); the plug member (14) having a tube body (141), the tube body (141) being disposed with a third flow channel (1411), and a first end of the tube body (141) being inserted into insertion hole (132), so that the second flow channel (132) communicating with the third flow channel (1411), and a blocking part (1412) being disposed inside the third flow channel (1411) to close the third flow channel (1411);
at least one second medicine mixing device (20, 20A) comprising: a puncturing part (11), a needleless additive port (12), an output part (13), a plug member (14), and a snap-on closed needleless connector module (21, 21A) as claimed in claim 1, and a first end of the puncturing part (11) being a sharp end; a U-shaped through hole (111) and at least one side hole (112) being disposed on one side wall of the puncturing part (11); a distance (D1) from the top of the U-shaped through hole (111) to the sharp end of the puncturing part (11) being less than a distance (D2) from the top of the at least one side hole (112) to the sharp end of the puncturing part (11); the puncturing part (11) being divided by a first partition (113) to divide an internal space into a first long hole (114) and at least a second long hole (115); the first long hole (114) and the U-shaped through hole (111) communicating with each other, the at least one second long hole (115) communicating with the at least one side hole (112); the needleless additive port (12) comprising a coupling seat (121), the coupling seat (121) being integrally formed on one side of the second end of the puncturing part (11) and being disposed with a first flow channel (1211), and the first flow channel (1211) communicating with the U-shaped through hole (111); the output part (13) being disposed at the second end of the puncturing part (11) and being disposed with a second flow channel (131) and an insertion hole (132), the second flow channel (131) communicating between the at least one second long hole (115) and the insertion hole (132), wherein a second partition (15) being disposed between the needleless additive port (12) and the output part (13) to separate the first flow channel (1211) and the second flow channel (131), and the first partition (113) being connected to the second partition (15), the plug member (14) having a tube body (141), the tube body (141) being disposed with a third flow channel (1411), a first end of the tube body (141) being inserted into the insertion hole (132), so that the second flow channel (131) and the third flow channel (1411) communicating with each other, and a blocking part (1412) being disposed inside the third flow channel (1411) to close the third flow channel (1411); the snap-on closed needleless connector module (21, 21A) as mentioned above being defined as a first closed needleless connector module (21, 21A), and the first coupling part (222) of the first closed needleless connector module (21, 21A) being used for coupling to a vial bottle (100A) or a syringe (200), and the second coupling part (232) of the first closed needleless connector module (21, 21A) being used for coupling to the coupling seat (121);
a first end of one of the at least two infusion drip sets (30, 30A) passing through the blocking part (1412) of the first medicine mixing device (10), thereby allowing one of the infusion drip sets (30, 30A) to communicate with the second flow channel (131) through the third flow channel (1411) of the first medicine mixing device (10), and a first end of the other one of the infusion drip sets (30, 30A) passing through the blocking part (1412) of the at least one second medicine mixing device (20, 20A), thereby allowing the other one of the infusion drip sets (30, 30A) to communicate with the second flow channel (131) through the third flow channel (1411) of the at least one second medicine mixing device (20, 20A);
a dispenser (40), comprising: a gathering part (41), at least two input terminals (42, 43), an output terminal (45), a snap-on closed needleless connector module (21B) as claimed in claim 1, and a screw-type needleless connector module (46); the input terminals (42, 43) being connected to a first end of the gathering part (41), the output terminal (45) being connected to a second end of the gathering part (41), the snap-on closed needleless connector module (21B) being defined as a second closed needleless connector module (21B), the first coupling part (222) of the second closed needleless connector module (21B) being used for coupling to a second end of one of the infusion drip sets (30, 30A), and the second coupling part (232) of the second closed needleless connector module (21B) being used for coupling to one of the input terminals (42, 43); the screw-type needleless connector module (46) comprising a screw-coupling part (461) and a second needleless connector (23A); the screw-coupling part (461) being disposed on the second end of sleeve (2211) of the other one of the infusion drip sets (30, 30A), a first end of the second needleless connector (23A) being screwed to the screw-coupling part (461), and a second end of the second needleless connector (23A) being disposed on the other one of the input terminals (42, 43);
a separator (50), comprising: a valve body (51), a screw-coupling structure (52), an output terminal (53), a third needleless connector (23B), and a snap-on closed needleless connector module (21C) as claimed in claim 1; an internal thread (521) of a first end of the screw-coupling structure (52) being screwed to an external thread (511) of a first end of the valve body (51) and defined as an input terminal of the separator (50); a first end of the output terminal (53) being disposed on a second end of the valve body (51); the third needleless connector (23B) being disposed at a third end of the valve body (51); the snap-on closed needleless connector module (21C) being defined as a third closed needleless connector module (21C), and the second coupling part (232) of the third closed needleless connector module (21C) being used for coupling to the screw-coupling structure (52); and
a retention needle (60), being disposed at a second end of the output terminal (53) of the separator (50).
